(19) Europäisches Patentamt
European Patent Office
Office européen des brevets

(11) Veröffentlichungsnummer: **0 293 931 B1**

(12) **EUROPÄISCHE PATENTSCHRIFT**

(45) Veröffentlichungstag der Patentschrift: **13.04.94**

(51) Int. Cl.$^5$: **C12Q 1/42**

(21) Anmeldenummer: **88108944.5**

(22) Anmeldetag: **03.06.88**

Die Akte enthält technische Angaben, die nach dem Eingang der Anmeldung eingereicht wurden und die nicht in dieser Patentschrift enthalten sind.

# (54) Verwendung von derivatisierter alkalischer Phosphatase als Standard.

(30) Priorität: **04.06.87 DE 3718753**

(43) Veröffentlichungstag der Anmeldung:
**07.12.88 Patentblatt 88/49**

(45) Bekanntmachung des Hinweises auf die Patenterteilung:
**13.04.94 Patentblatt 94/15**

(84) Benannte Vertragsstaaten:
**AT BE CH DE ES FR GB GR IT LI LU NL SE**

(56) Entgegenhaltungen:
**EP-A- 0 130 708**
**WO-A-84/02720**
**US-A- 4 681 842**

**DAS ÄRZTLICHE LABORATORIUM, vol. 32, 1986; W. BEHR et al., Seiten 159-165&NUM;**

**KLINISCHE WOCHENSCHRIFT, vol. 58, 1980; Seiten 947-951&NUM;**

**CLINICAL CHEMISTRY, vol. 28, 1982; Seiten 2007-2016&NUM;**

(73) Patentinhaber: **BOEHRINGER MANNHEIM GMBH**

**D-68298 Mannheim(DE)**

(72) Erfinder: **Tischer, Wilhelm, Dr.**
**Finkenweg 5**
**D-8123 Peissenberg(DE)**
Erfinder: **Gerber, Martin, Dr.**
**Ignaz Manz-Strasse**
**D-8120 Weilheim-Unterhausen(DE)**
Erfinder: **Vetter, Hellmuth, Dr.**
**Kreuzeckstrasse 17**
**D-8132 Tutzing(DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

**EP 0 293 931 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung einer derivatisierten alkalischen Phosphatase als Standard für die Bestimmung von humaner alkalischer Phosphatase.

Die alkalische Phosphatase (EC 3.1.3.1.), ein Enzym, das Ester der Phosphorsäure hydrolysiert, wird im Körper im wesentlichen in der Leber, in den Knochen, in der Niere, im Dünndarm, sowie bei Schwangeren auch in der Placenta gebildet. Die einzelnen Isoenzyme unterscheiden sich vor allem in ihrem Kohlenhydratanteil. Bei Erkrankungen der Organe gelangt das in diesen Organen jeweils gebildete Isoenzym vernehrt in den Blutkreislauf. Der Nachweis der einzelnen Enzyme im Serum ist daher diagnostisch sehr wertvoll. Der Anteil an Nieren-, Dünndarm- und Placentaphosphatase im Serum ist in der Regel gering. Darüberhinaus lassen sich Dünndarm- und Placentaphosphatase leicht von den übrigen Isoenzymen abtrennen durch übliche Trennverfahren wie Chromatographie. Vor allem interessant ist die Differenzierung nach im Knochen gebildeter oder in der Leber gebildeter alkalischer Phosphatase. Auf diese Weise sind Hinweise auf Karzinome, auf Knochenerkrankungen und Art und Stadium von Lebererkrankungen zu erhalten. Es ist daher im Bereich der klinischen Diagnostik wichtig, nicht nur die Gesamtkonzentration der alkalischen Phosphatase im Blut, sondern auch den Anteil der beiden Isoenzyme, zu bestimmen. Eine Möglichkeit hierzu bietet das verschiedene Verhalten der beiden Isoenzyme gegenüber Weizenkeimlectin. Während die alkalische Knochenphosphatase mit Weizenkeimlectin sofort reagiert und ausfällt, bleibt die Leberphosphatase länger in Lösung und bildet erst langsam mit Weizenkeimlectin unlösliche Komplexe.

Es wird nun in Ärztl. Lab. 32, 159-165 (1986) ein Verfahren zur quantitativen Bestimmung der alkalischen Gesamtphosphatase sowie der alkalischen Knochenphosphatase beschrieben, bei dem zuerst in einer Probe der Gesamtgehalt der alkalischen Phosphatase bestimmt wird. Anschließend wird mit Weizenkeimlectin die alkalische Knochenphosphatase ausgefällt und im Überstand der Anteil der alkalischen Leberphosphatase bestimmt. Aus diesen beiden Werten läßt sich dann in einfacher Weise der Anteil der alkalischen Knochenphosphatase ermitteln.

Ein Problem bei dieser Bestimmungsmethode besteht darin, daß keine Standardlösungen zur Verfügung stehen. Da die Bestimmung des Anteils der alkalischen Knochenphosphatase nach einer Ausfällungsreaktion erfolgt, wäre es für die Genauigkeit der Bestimmung wesentlich, in einer Vergleichslösung, deren Gehalt an den einzelnen Isoenzymen bekannt ist, zu ermitteln, welcher Anteil der Isoenzyme in welcher Fraktion zu finden ist. Für genaue Bestimmungen ist daher die Bereitstellung einer Standardlösung wünschenswert.

Bei den bisher handelsüblichen Tests wird zur Kontrolle nur eine Phosphatase eingesetzt, die nicht mit Weizenkeimlectin fällbar ist. Dies ist von Nachteil, da gerade die Ausfällung der Phosphatase Fehlermöglichkeiten beinhaltet, die durch Überprüfung mit einer Standardlösung ermittelt werden sollen. Ein weiteres Problem besteht darin, daß für die Standardlösung eine Phosphatase zur Verfügung gestellt werden muß, deren Aktivität der im Blut nachzuweisenden Phosphatase entspricht.

Da die aus den Organen extrahierten Isoenzyme mit den entsprechenden Isoenzymen im Serum nicht identisch sind und daher ein anderes Fällungsverhalten gegenüber Weizenkeimlectin zeigen und da für die Anreicherung aus Serum nicht genügend große Mengen zur Verfügung stehen, war es Aufgabe der vorliegenden Erfindung, eine Standardlösung für die Verwendung der differenzierten Bestimmung von alkalischer Phosphatase bereitzustellen.

Diese Aufgabe wird gelöst durch die Verwendung von alkalischer Phosphatase, an die ein Kohlenhydrat der folgenden Formel:

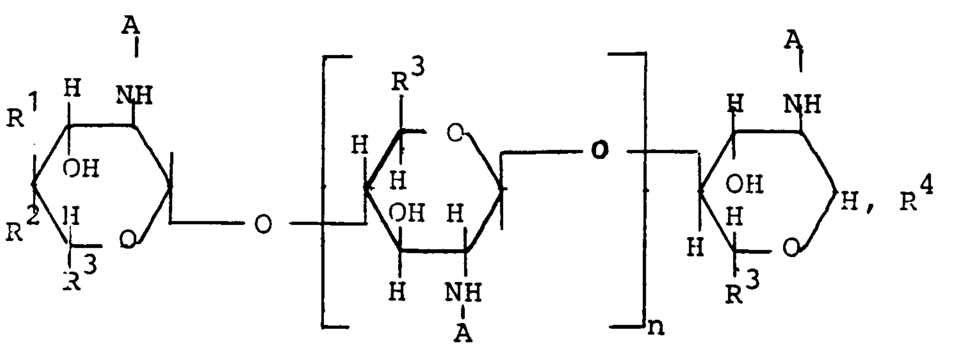

(I)

worin n eine ganze Zahl von 0 bis 3, A eine Acylgruppe mit 2 bis 5 C-Atomen, $R^1$ und $R^2$ jeweils H oder OH, $R^3$ -COOH oder $-CH_2OH$ und $R^4$-OH, $-CHOH-CHOH-CH_2OH$,

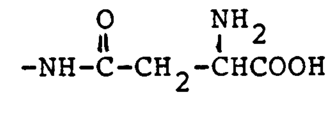

bedeuten kann oder ein ein Kohlenhydrat der Formel (I) enthaltender Komplex, kovalent gebunden ist, als Standard für die Bestimmung von gesamter humaner alkalischer Phosphatase und für die Bestimmung von humaner alkalischer Knockenphosphatase nach Fällung mit Weizenkeimlectin.

Bei Verwendung der erfindungsgemäß zur Verfügung gestellten derivatisierten alkalischen Phosphatase gelingt es, das Fällungsverhalten der in Serum vorkommenden alkalischen Knochenphosphatase nachzubilden, ohne daß Aktivitätsverluste entstehen.

Erfindungsgemäß wird eine alkalische Phosphatase verwendet, die nicht mit Weizenkeimlectin fällbar ist. Hier kommen insbesondere tierische Phosphatasen sowie die alkalische Phosphatase aus Humanplacenta in betracht. Bevorzugt wird das Isoenzym aus Kälberdarm oder aus Humanplacenta verwendet.

Diese nicht fällbare alkalische Phosphatase wird dann derivatisiert mit einem Kohlenhydrat. Als Kohlenhydrat wird eine Verbindung der folgenden Formel:

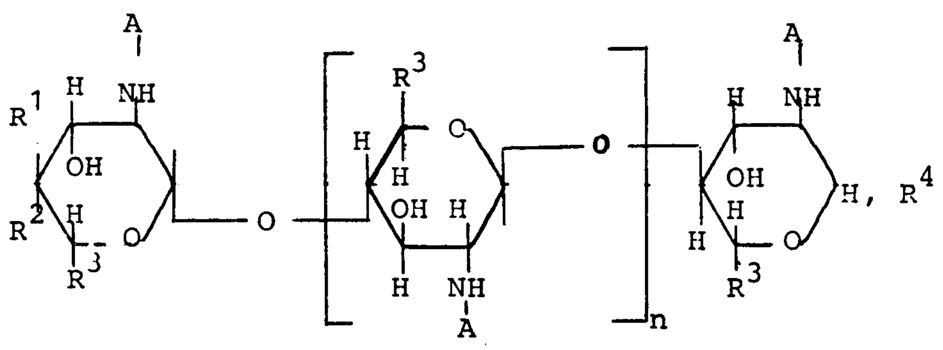

(I)

worin n eine ganze Zahl von 0 bis 3, A eine Acylgruppe mit 2 bis 5 C-Atomen, $R^1$ und $R^2$ jeweils H oder OH, $R^3$ -COOH oder $-CH_2OH$ und $R^4$-OH, $-CHOH-CHOH-CH_2OH$,

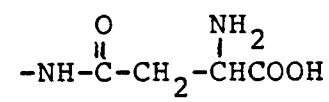

bedeuten kann oder ein ein Kohlenhydrat der Formel (I) enthaltender Komplex verwendet.

Bevorzugt werden Verbindungen verwendet, bei denen A ein Acetylrest ist.

Dieses Kohlenhydrat wird an die alkalische Phosphatase kovalent gebunden. Dieses Kohlenhydrat vermittelt die selektive Fällbarkeit mit Weizenkeimlectin.

Zur Derivatisierung der Phosphatase kann die Verbindung der allgemeinen Formel (I) verwendet werden. Ebenfalls möglich ist es, Protein-Polysaccharid-Komplexe oder Polysaccharide zu verwenden, die eine Struktur gemäß Formel (I) enthalten. So können Komplexe verwendet werden, die aus Verbindungen gemäß Formel (I) bestehen, an deren OH-Gruppen weitere Zucker oder Proteine gebunden sind.

Bevorzugt wird als Kohlenhydrat das als Chitotriose bezeichnete Tri-(N-acetyl-glucosamin), das als Chitobiose bezeichnete Di-(N-acetyl-glucosamin) oder ein Trisaccharid aus zwei Molekülen N-acetyl-glucosamin und einem Molekül N-Acetyl-neuraminsäure oder -neuraminose verwendet. Weiterhin bevorzugt wird das aus Eiklar gewonnene Glykoprotein Ovomucoid. Besonders bevorzugt wird Chitotriose oder Ovomucoid verwendet.

Das Kohlenhydrat wird an die alkalische Phosphatase kovalent gebunden. Verfahren zur kovalenten Bindung von Polysacchariden an Proteine bzw. zur kovalenten Bindung von Proteinen mit Proteinen sind an sich bekannt und dem Fachmann sind die jeweils geeigneten Verfahren geläufig. In der Regel wird ein Kohlenhydrat durch eine chemische Reaktion aktiviert, wodurch eine chemische Bindung an Amino-,

Hydroxyl- oder Carboxylgruppen von Proteinen ermöglicht wird. Solche Aktivierungen sind dem Fachmann geläufig und sind beispielsweise in Advances in Carbohydrate Chemistry and Biochemistry, Academic Press, (1980), Vol. 37, Seiten 225 bis 281 beschrieben. Man kann statt dessen reaktive Gruppen von Kohlenhydraten wie z. B. Aminogruppen oder Carboxylgruppen, die gegebenenfalls vorher in das Kohlenhydrat eingeführt wurden, verwenden, um diese mit reaktiven Gruppen von Proteinen unter Zusatz von bifunktionellen Reagenzien miteinander chemisch zu verknüpfen. Solche bifunktionellen Reagenzien sind z. B. die im Katalog von Pierce Chemical Company (NL) (1987), Seiten 311 bis 349 beschriebenen Cross-Linking Reagents. Bevorzugt werden als bifunktionelle Verbindungen homobifunktionelle Verbindungen wie Imidoester oder N-Hydroxysuccinimidester oder heterobifunktionelle Verbindungen, die mindestens eine N-Hydroxysuccinimidester-, -sulfhydryl-, -maleinimid-, -pyridyldisulfid- oder -halogengruppe enthalten, verwendet. Ebenso geeignet ist die Verwendung von Glutardialdehyd.

Für die Ausfällung des erfindungsgemäß derivatisierten Isoenzyms wird eine Lösung von Weizenkeimlectin verwendet. Das Fällungsverhalten der Phosphatase ist abhängig von der Anzahl und der Affinität der Bindungsstellen an Phosphatase und Weizenkeimlectin. Die jeweils günstigste Konzentration an Weizenkeimlectin wird daher jeweils abhängig von dem verwendeten Derivat der Phosphatase bestimmt.

Die erfindungsgemäß derivatisierte alkalische Phosphatase kann sehr vorteilhaft als Standard für Bestimmungen von alkalischer Knochenphosphatase neben alkalischer Leberphosphatase verwendet werden. Die Standardlösungen werden dabei in an sich bekannter Weise hergestellt und für das Bestimmungsverfahren eingesetzt. Die Bestimmung der alkalischen Phosphatase erfolgt in der Regel über die Farbänderung von Substraten oder Dichtebestimmungen.

Die Erfindung wird noch durch die folgenden Beispiele erläutert:

**Beispiel 1**

Es wurde eine mit Ovomucoid vernetzte alkalische Phosphatase hergestellt. Dazu wurden 20 ml einer Lösung von 200 mg Type III-O: Chicken egg white purified ovomucoid mit einem Molekulargewicht von ungefähr 28.000 (T 2011 Sigma) in bidestilliertem Wasser mit 10 ml Kälberdarm-AP, enthaltend 100 mg Phosphatase mit einer Aktivität von etwa 66 kU, versetzt. Eine 25%ige Glutardialdehydlösung wurde 1:10 mit einer Lösung, die 1 mol/l Triethanolamin, pH 7,0, 1 mmol/l $MgCl_2$ und 0,1 mmol/l $ZnCl_2$ enthielt, verdünnt. 3,0 ml dieser Glutardialdehydlösung wurden dem Ansatz zugesetzt. Die Lösung wurde dann 6 Stunden bei +4°C gerührt und anschließend 18 Stunden bei +4°C inkubiert. Die Reaktion wurde dann durch Zusatz von 0,6 ml einer 1M Lysin HCl-Lösung mit einem pH von 6,0 abgebrochen. Anschließend wurden der Lösung 600 mg Ficoll 70 zugegeben. Die entstandene Lösung wurde 24 Stunden lyophilisiert. Es wurden ca. 3150 mg Lyophilisat erhalten mit einer Aktivität von 8,3 U/mg.

**Beispiel 2**

40 mg N, N′, N″-Triacetylchitotriose (T 2144, Sigma) wurden in 1 ml 0,8 mmol/l Acetat, pH 5,5 gelöst und auf +3°C abgekühlt. Anschließend wurden 13,3 mg $NaJO_4$ zugegeben und es wurde 40 Minuten bei +3°C inkubiert. Nach einer weiteren Zugabe von 182 µl Ethylenglykol wurde die Temperatur auf 22°C erhöht und 20 Minuten inkubiert. Anschließend wurde mit 1 mmol/l Carbonat/Bicarbonat-Pufferlösung pH 9,8 der pH-Wert des Ansatzes auf pH 8,8 eingestellt. Daraufhin wurde portioniert 0,8 ml einer 2,2 mg/ml enthaltenden alkalischen Phosphataselösung zugegeben. Es wurde so verfahren, daß jeweils nach 30 Minuten 8 mg der mit $NaJO_4$ oxidierten N,N′,N″-Triacetylchitotriose zur alkalischen Phosphatase gegeben wurde. Nach Zugabe von 40 mg der oxidierten Chitotriose wurde die Kupplung abgebrochen. Der pH-Wert wurde mit 1 mmol/l Carbonat/Bicarbonat-Puffer, pH 9,8 auf einem pH-Wert von 8,8 gehalten. Die Kupplung erfolgte bei Raumtemperatur. Anschließend wurde mit 1 mmol/l Triethanolaminpuffer (TRA) pH 7,0 der pH-Wert des Ansatzes auf 8,0 eingestellt. Es wurden 13,3 mg $NaBH_3CN$ und 532 µl einer Glycinlösung (250 mg/ml) zugegeben, wobei der pH-Wert mit TRA-Puffer auf 8,0 gehalten wurde. Es wurde 15 Minuten bei Raumtemperatur reagieren gelassen. Der Ansatz wurde über Nacht bei +4°C gegen 5 l 10 mM TRA pH 7,6 dialysiert. Es wurde derivatisierte Phosphatase mit einer Aktivitätsausbeute von 64 % erhalten.

**Beispiel 3**

Es wurde alkalische Phosphatase mit Ovomucoid derivatisiert, gemäß dem in Beispiel 1 beschriebenen Verfahren. Als Phosphatase wurde jedoch alkalische Phosphatase aus Humanplacenta (P 3895, Sigma) verwendet. Die Aktivitätsausbeute betrug 28 %.

**Beispiel 4**

Es wurde eine Phosphatase derivatisiert, wie im Beispiel 1 beschrieben. Als Ausgangsphosphatase wurde jedoch alkalische Phosphatase aus E. coli (P4151, Sigma) verwendet. Die Aktivitätsausbeute betrug 37%.

**Beispiel 5**

Es wurden die in den vorherigen Beispielen erhaltenen derivatisierten Phosphatasen auf ihre Fällbarkeit mit Weizenkeimlectin getestet.

Prinzip:

Es wird eine Verdünnungsreihe des Weizenkeimlectins (WGA) hergestellt und danach wird das WGA jeweils 1 + 1 mit modifizierter Kälberdarm-AP in Humanserummatrix gemischt. Die AP fällt dabei aus, sie wird abzentrifugiert und im Überstand wird die restliche AP-Aktivität bestimmt. Diese Aktivitäten werden bezogen auf die Restaktivität mit der Lectinkonzentration 0 mg/ml. Durch eine Auftragung der prozentualen Werte gegen die eingesetzte Lectinkonzentration läßt sich eine Fällungskurve zeichnen (Fig. 1). Als optimale Lectinkonzentration hat sich die Konzentration erwiesen, die etwa 20 % Restaktivität ergibt (Probenablesung).

Reagenzien

Puffer: 5 mM Acetatpuffer pH = 5,0
AP-Probe: ca. 1000 U/l (25 °C) modifizierte Kälberdarm-AP in Tris-Puffer, pH 7,0, mit 0,02 mM Zinkchlorid und 1 mM Magnesiumchlorid und 1% Rinderserumalbumin.

Die Bestimmung der AP-Aktivität erfolgte nach dem BM-AP-Test (Katalogbestellnummer 415278, Boehringer Mannheim GmbH).

Dem Test liegt die folgende Reaktion zugrunde:

$$\text{4-Nitrophenylphosphat} + H_2O \xrightarrow{\text{alkalische Phosphatase}} \text{4-Nitrophenol} + Pi$$

Die Zunahme der Absorption wird bei Hg 405 nm gemessen.

Als Reagenzien werden verwendet:
Diethanolaminpuffer (1 mol/l; pH 9,8; $MgCl_2$ 0,5 mmol/l). 4-Nitrophenylphosphat (0,67 mol/l): 250 mg 4-Nitrophenylphosphat-$Na_2$ pro 1 ml redist. Wasser.

In einer Küvette werden 3 ml Pufferlösung und 0,05 ml 4-Nitrophenylphosphatlösung gemischt und bei einer Temperatur von 37 °C inkubiert. Die Reaktion wird gestartet durch Zugabe von 0,05 ml Probe. Der Anstieg der Absorption (ΔA) pro Minute wird gemessen, wobei der lineare Anteil der Kurve ausgewertet wird. Zum Vergleich wird ein Leerwert bestimmt, wobei statt der Probe eine Magnesiumchloridlösung verwendet wird.

Aus dem Anstieg der Absorption (ΔA) kann dann die Volumenaktivität berechnet werden nach der Formel

$$\frac{3,10}{18,2 x 0,05 x 1} \text{ x } \Delta A/\text{min } [\text{U/ml Probelösung}]$$

Durchführung:

Verdünnungsreihe: Das zu testende Lectin wird im Acetatpuffer verdünnt zu ca. 10 Konzentrationen. Folgende Konzentrationen haben sich als günstig erwiesen:

0 mg/ml; 0,1 mg/ml; 0,5 mg/ml; 1 mg/ml; 2 mg/ml; 3 mg/ml; 4 mg/ml; 5 mg/ml; 7,5 mg/ml; 10 mg/ml;

20 mg/ml; 30 mg/ml.

Fällung: Jeweils 0,1 ml der Lectinverdünnungen werden mit 0,1 ml des AP-Serums gemischt und bei 37 °C 1/2 Stunde inkubiert. Danach werden die Mischungen ca. 2 Minuten zentrifugiert und der Überstand wird weiter verwendet.

AP-Bestimmung: In den Überständen wird die AP-Aktivität mit dem AP-Test optimiert bei 25 °C nach Arbeitsanleitung bestimmt.

Auswertung: Die gemessenen Aktivitäten werden auf die Aktivität mit der Lectinkonzentration 0 mg/ml bezogen und als Prozentwerte angegeben. Es erfolgt eine Auftragung der Restaktivitäten als Funktion der eingesetzten Lectinkonzentration.

Ergebnis: Fig. 1 zeigt die erhaltenen Fällungskurven für die alkalische Phosphatase aus Kälberdarm, die entsprechend der Beispiele 1 bis 4 modifiziert wurde. Die Quervernetzung mit Ovomucoid brachte hervorragende Fällbarkeiten mit niedrigen WGA-Konzentrationen, während alle anderen Verfahren APs lieferten, die nur mit höheren WGA-Konzentrationen eine Fällung durch das Lectin ermöglichten.

Bewertung: Ziel der Aufreinigung sollte es sein, entweder möglichst kleine Konzentrationen an WGA zur 20%igen Restaktivität zu erhalten, oder aber die Bedingungen der Aufreinigung so zu optimieren, daß immer 5 mg/ml WGA erreicht werden.

## Patentansprüche

1. Verwendung von alkalischer Phosphatase, an die ein Kohlehydrat der allgemeinen Formel:

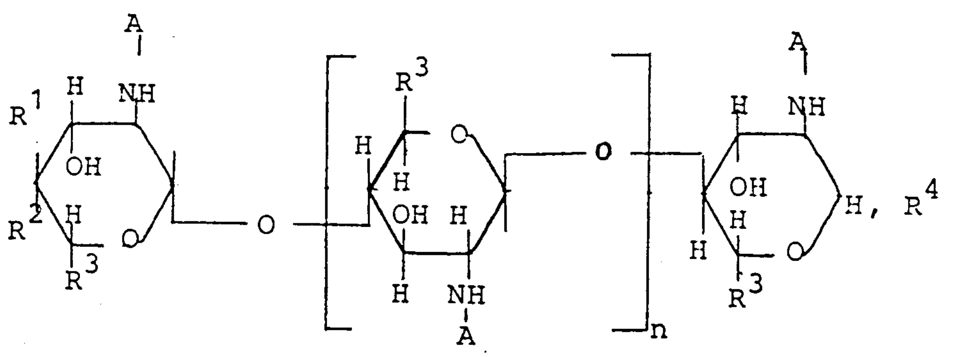

(I)

worin n eine ganze Zahl von 0 bis 3, A eine Acylgruppe mit 2 bis 5 C-Atomen, $R^1$ und $R^2$ jeweils H oder OH, $R^3$ -COOH oder $-CH_2OH$ und $R^4$-OH, $-CHOH-CHOH-CH_2OH$,

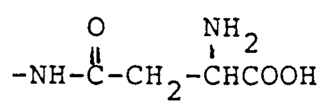

bedeuten kann oder ein ein Kohlenhydrat der Formel (I) enthaltender Komplex, kovalent Gebunden ist, als Standard für die Bestimmung von gesamter humaner alkalischer Phosphatase und für die Bestimmung von humaner alkalischer Knochenphosphatase nach Fällung mit Weizenkeimlectin.

2. Verwendung nach Anspruch 1, **dadurch gekennzeichnet,** daß als Kohlenhydrat Ovomucoid, Chitobiose, Chitotriose und/oder N-Acetylglucosamin-n-acetyl-neuraminsäure verwendet wird.

3. Verwendung nach Anspruch 1 oder 2, **dadurch gekennzeichnet,** daß die Acylgruppe eine Acetylgruppe ist.

4. Verwendung nach einem der vorhergehenden Ansprüche, **dadurch gekennzeichnet,** daß das Kohlenhydrat an die alkalische Phosphatase über eine bifunktionelle Verbindung gebunden ist.

5. Verwendung nach Anspruch 4, **dadurch gekennzeichnet,** daß die bifunktionelle Verbindung ein homobifunktioneller Imidoester oder N-Hydroxysuccinimidester ist.

6. Verwendung nach Anspruch 4, **dadurch gekennzeichnet,** daß die bifunktionelle Verbindung Glutardialdehyd ist.

7. Verwendung nach Anspruch 4, **dadurch gekennzeichnet,** daß die bifunktionelle Verbindung eine heterobifunktionelle Verbindung ist, die mindestens eine N-Hydroxysuccinimidester-, -sulfhydryl-, -maleinimid-, -pyridyldisulfid- oder -halogengruppe enthält.

8. Verwendung nach einem der vorhergehenden Ansprüche, **dadurch gekennzeichnet,** daß als alkalische Phosphatase eine aus Kälberdarm oder Humanplacenta gewonnene alkalische Phosphatase verwendet wird.

## Claims

1. Use of alkaline phosphatase to which is covalently bound a carbohydrate of the general formula:

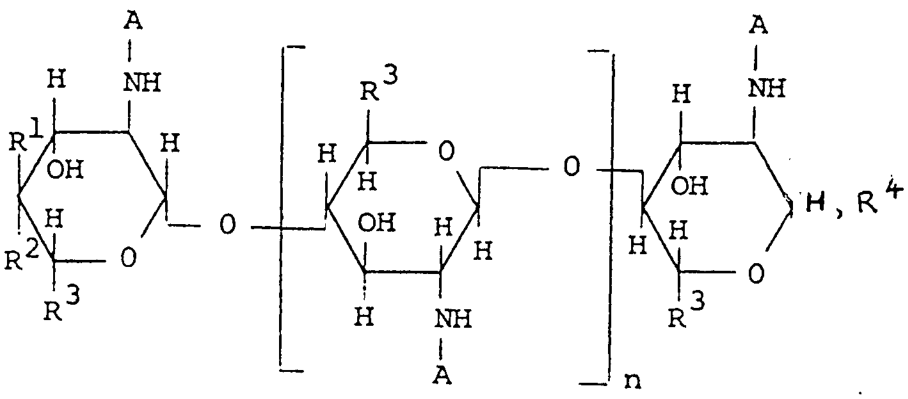

(I)

wherein n can signify a whole number of 0 to 3, A an acyl group with 2 to 5 C-atoms, $R^1$ and $R^2$ in each case H or OH, $R^3$ -COOH or $-CH_2OH$ and $R^4$ -OH, $-CHOH-CHOH-CH_2OH$,

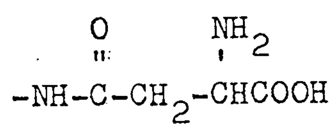

or a complex containing a carbohydrate of the formula (I), as standard for the determination of total human alkaline phosphatase and for the determination of human alkaline bone phosphatase after precipitation with wheat germ lectin.

2. Use according to claim 1, characterised in that ovomucoid, chitobiose, chitotriose and/or N-acetylglucosamine-N-acetylneuraminic acid is used as carbohydrate.

3. Use according to claim 1 or 2, characterised in that the acyl group is an acetyl group.

4. Use according to one of the preceding claims, characterised in that the carbohydrate is bound to the alkaline phosphatase via a bifunctional compound.

5. Use according to claim 4, characterised in that the bifunctional compound is a homobifunctional imido ester or N-hydroxysuccinimide ester.

6. Use according to claim 4, characterised in that the bifunctional compound is glutardialdehyde.

7. Use according to claim 4, characterised in that the bifunctional compound is a heterobifunctional compound which contains at least one N-hydroxysuccinimide ester, -sulphhydryl, -maleinimide, -pyridyldisulphide or -halogen group.

8. Use according to one of the preceding claims, characterised in that an alkaline phosphatase obtained from calf intestine or human placenta is used as alkaline phosphatase.

**Revendications**

1. Utilisation d'une phosphatase alcaline à laquelle est lié de manière covalente un glucide de formule générale:

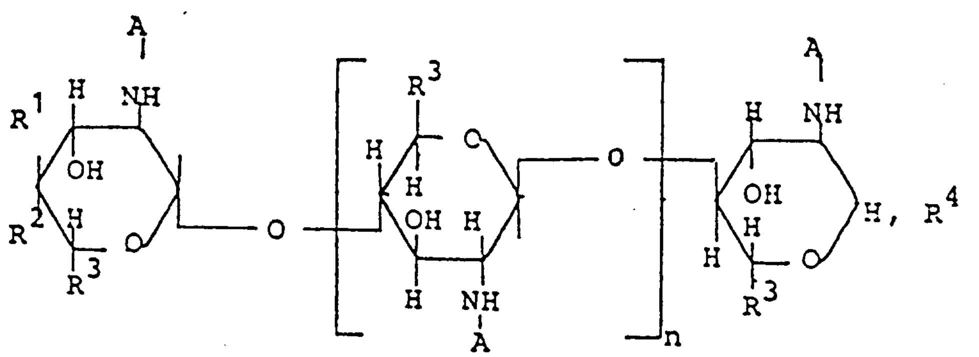

(I)

dans laquelle n peut représenter un nombre entier de 0 à 3, A peut représenter un groupe acyle de 2 à 5 atomes de carbone, $R^1$ et $R^2$ peuvent représenter chacun H ou OH, $R^3$ peut représenter -COOH ou $-CH_2OH$ et $R^4$ peut représenter -OH, $-CHOH-CHOH-CH_2OH$,

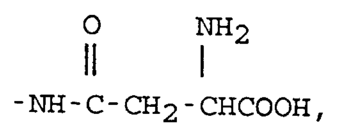

ou un complexe contenant un glucide de formule (I), comme standard pour la détermination de la phosphatase alcaline humaine totale et pour la détermination de la phosphatase alcaline humaine osseuse après précipitation avec la lectine de germes de blé.

2. Utilisation selon la revendication 1, caractérisée en ce que l'on utilise comme glucide l'ovomucoïde, le chitobiose, le chitotriose et/ou l'acide N-acétylglucosamine-N-acétylneuraminique.

3. Utilisation selon la revendication 1 ou 2, caractérisée en ce que le groupe acyle est un groupe acétyle.

4. Utilisation selon l'une des revendications précédentes, caractérisée en ce que le glucide est lié à la phosphatase alcaline par l'intermédiaire d'un composé bifonctionnel.

5. Utilisation selon la revendication 4, caractérisée en ce que le composé bifonctionnel est un imidoester ou ester de N-hydroxysuccinimide homobifonctionnel.

6. Utilisation selon la revendication 4, caractérisée en ce que le composé bifonctionnel est le dialdéhyde glutarique.

7. Utilisation selon la revendication 4, caractérisée en ce que le composé bifonctionnel est un composé hétérobifonctionnel qui contient au moins un groupe ester de N-hydroxysuccinimide, sulfydryle, maléimide, pyridyldisulfure ou halogène.

8. Utilisation selon l'une des revendications précédentes, caractérisée en ce que l'on utilise comme phosphatase alcaline une phosphatase alcaline obtenue à partir d'intestin de veau ou de placenta humain.

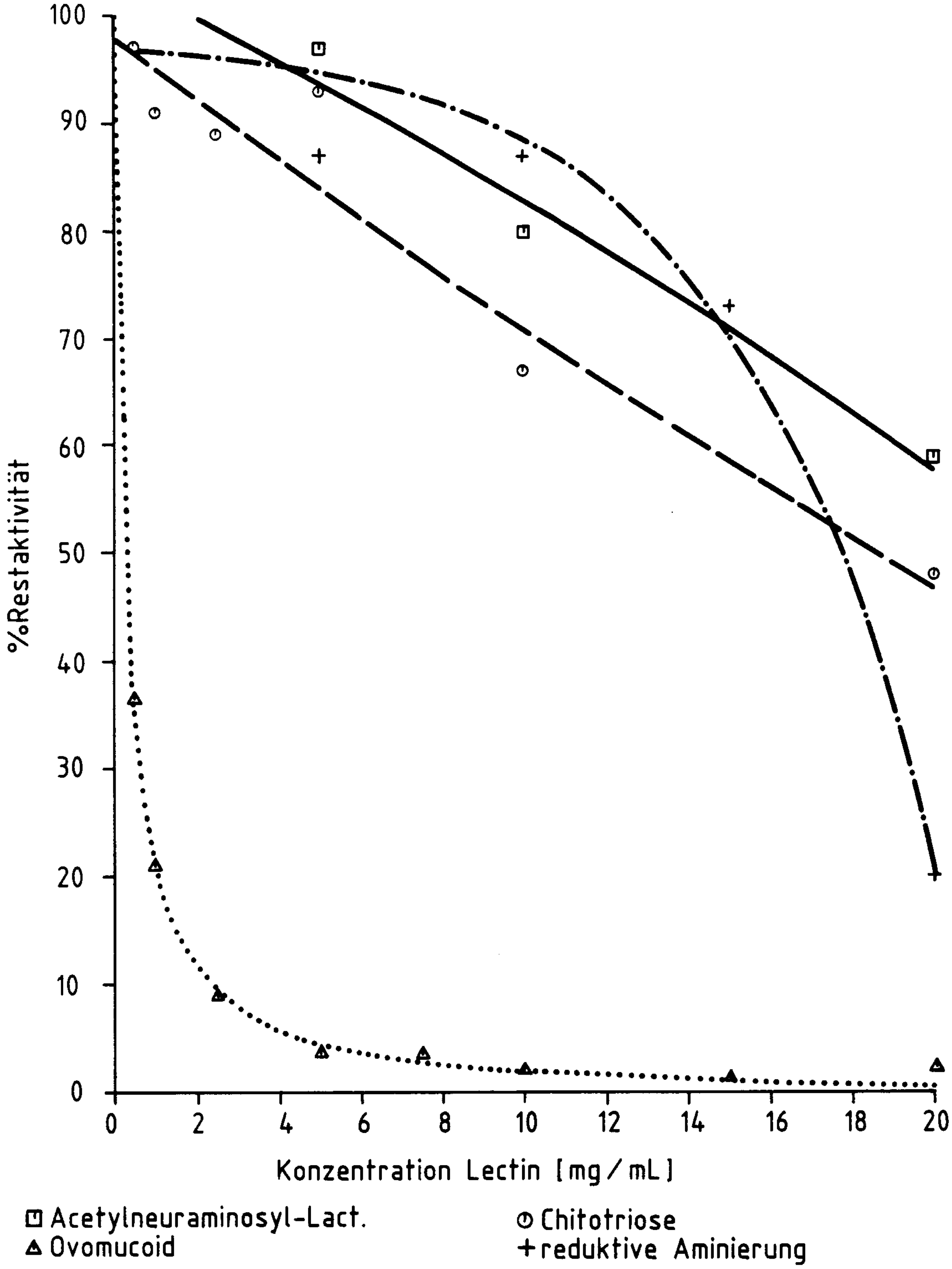
100
90
80
70
60
50
40
30
20
10
0
%Restaktivität
0
2
4
6
8
10
12
14
16
18
20
Konzentration Lectin [mg / mL]
Acetylneuraminosyl-Lact.
Chitotriose
Ovomucoid
reduktive Aminierung